# EUROPEAN PATENT SPECIFICATION

(11) **EP 1 047 360 B1**
(45) Date of publication and mention of the grant of the patent: **13.10.2004**
(21) Application number: 99902297.3
(22) Date of filing: 15.01.1999
(51) Int. Cl.: A61F 5/453

(54) **TWO-PIECE EXTERNAL INCONTINENCE DEVICE**
ZWEITEILIGE EXTERNE INKONTINENZVORRICHTUNG
DISPOSITIF D'INCONTINENCE EXTERNE EN DEUX PARTIES

(30) Priority: 15.01.1998 US 71519 P
(43) Date of publication of application: 02.11.2000
(73) Proprietor: Bioderm, Inc., St. Petersburg, FL 33716-4807 (US)
(72) Inventor: KAY, Dennis, M., Tampa, FL 33602 (US)
(74) Representative: Plougmann & Vingtoft A/S
(86) International application number: PCT/US1999/000923
(87) International publication number: WO 1999/036009

(56) References cited:
- GB-A- 2 197 849
- US-A- 3 835 857
- US-A- 4 013 077
- US-A- 4 563 183
- US-A- 4 776 848
- US-A- 5 263 947

## Description

### BACKGROUND OF THE INVENTION

The present invention relates to a two-piece external incontinence device for medical use. Urinary incontinence, a medical problem, is treated currently with invasive catheters, condom catheters, incontinence pads and diapers.

There are several problems associated with the conventional treatments. For example, indwelling invasive urinary catheters cause urinary tract infections. The presence of an invasive catheter in the urethra is thought to facilitate the infiltration and growth of microorganisms. Microorganisms at the catheter entrance site ascend the catheter through the urethra into the urinary tract, resulting in infections.

The conventional condom catheter is essentially a bag connected to a tube. The condom catheter design presents no barrier to the infiltration of microorganisms into the urethra. The sheath is usually attached to the penis by a circumferential adhesive band. Often the attachment is inadequate and the condom catheter falls off or leaks. In other cases, the circumferential adhesive band results in irritation or even strangulation of the penis. Inside the condom or bag, the moist environment is ideal for microorganism growth. Microorganisms growing in the moist environment in the condom can enter the urethra and proceed into the urinary tract causing urinary tract infections.

Incontinence pads or diapers, whether used on male or female patients, also contribute to the onset and severity of infections. Excreted urine and/or feces contained in these devices are excellent media for bacterial growth. Bacteria can enter the urethra because in long term care facilities personnel are often unable to change patients' diapers as frequently as recommended by good medical practice.

Another problem associated with current incontinence treatments, particularly true of the condom catheter, is skin maceration and irritation. The epidermis is composed of dead cells which protect the underlying living dermis cells from physical and chemical irritation and from microbial invasion. Maceration refers to the problems associated with prolonged exposure of the dermis to moisture. When epidermal cells are exposed to moisture for a sufficient period of time, they absorb water, swell, and slough off, thus exposing the physiologically active dermal layer to whatever chemical or microbial entities are present in the immediate environment.

Maceration occurs not only when the skin is exposed to water from external sources but also when moisture is trapped against the skin surface by a water-impermeable membrane. Skin continually emits water, particularly in the genital area. If the emitted water is trapped against the skin surface by a water-impermeable membrane and is not able to evaporate, then maceration occurs.

If maceration proceeds unabated, an inflammation of cellular and connective tissue, caused by the failure of the epidermis, may occur. This infiltration is often accompanied or followed by microbial invasion, causing a cellulitis or tissue infection. Microbial invasion causes further tissue inflammation and destruction. In severe cases of cellulitis, extensive tissue destruction, necrosis or gangrene ensues, necessitating removal of the gangrenous tissues or the entire affected limb.
Maintaining skin dryness is necessary to prevent the onset of maceration and further degenerative states.

The condom catheter does not allow the necessary evaporation of moisture from the skin surface. Not only do the walls of the condom trap the emitted moisture against the skin surface, but urination also adds to the moisture trapped inside the condom and contributes chemical irritants, such as urea, to the enclosed area. Thus, conventional condom catheters cause maceration and skin irritation.

The maceration and skin irritation caused by condom catheters make them physically uncomfortable, and patients routinely remove them, either deliberately or inadvertently. In addition, macerated tissue is moist and loose. Adhesives, therefore, do not adhere well to macerated skin. Thus, simply adding adhesive to the condom catheter does not help secure the condom catheter in place. The condom catheter becomes loose or falls off of the patient due to maceration under the adhesive.

Commonly owned United States Patent No. 5,263,947 discloses a newer external incontinence device according to the preamble of claim 1. The patent describes a one-piece device having a housing with an outlet conduit, a plurality of leaves having a thin, vapor permeable adhesive on their interior surface, a microbial barrier layer provided on an inner surface of the housing, and a barrier disc provided on a rear surface of the housing. This device reduces or eliminates skin maceration, skin irritation, microbial skin infiltration and infection, and urinary tract infection associated with conventional urinary catheters and incontinence devices. Although successful in avoiding some problems experienced with conventional treatments, this device may also be improved, as will be described below.

The difficulties mentioned above with respect to conventional treatments are among many that reduce the effectiveness and safety of external incontinence devices. Other noteworthy problems also may exist; however, those presented here are sufficient to demonstrate that conventional treatments in this regard admit to some manner of improvement.

### SUMMARY OF THE INVENTION

Accordingly, it is a general object of the invention to provide an external incontinence device which will minimize or eliminate microbial infiltration of microorganisms into the urethra, thus reducing the incidence of urinary tract infections associated with use of conventional urinary catheters, incontinence pads and diapers.

It is a specific object of the invention to provide an external incontinence device which does not suscept the patient to skin maceration and irritation, thereby eliminating microbial invasion which can cause cellulitis or tissue infection.

It is a further object of the invention to provide an external incontinence device which does not trap moisture emitted by the skin or collect urine so that proper adhesion to the skin surface can occur.

It is yet a further object of the present invention to reduce the time and the skill necessary for the application of an external incontinence device to a patient, whether this device is self-applied by the patient or applied by a health care worker such as a nurse, physician, or nursing assistant.

It is still a further object of the present invention to provide safety and protection to the person wearing the device such that if the patient purposefully or inadvertently pulls on the drainage tube, the pulling force will not be transferred to the patient.

It is an additional object of the present invention to permit intermittent catheterization of the urethra and bladder through the device while the device remains in place on the patient.

It is yet still another object of the present invention to provide an easier procedure for a patient wearing the device to switch between two collection devices, as, for example, between a urinary leg bag and the often larger-volume night drainage bag or bottle.

It is yet still a further object of the present invention to provide a device which allows the individual applying the device to more easily and accurately align the outlet conduit with the meatus.

One preferred embodiment of the invention which is intended to accomplish at least some of the foregoing objects includes a first member, a second member dimensioned to releasably engage the first member, a barrier disc attached to the first member, and a wafer seal that allows for optimal fit for a wide variety of anatomical variations and sizes. The first member of the device attached to the patient, and the second member of the device attaches to standard medical tubing of drainage containers. Because the first and second members are releasable from each other, the device accomplishes objects of the invention.

The barrier disc is provided on a first surface of the first member, in context, closest to the patient. The barrier disc will adhere to, and create a seal between, the patient's skin and the relatively soft first member. The first member and the barrier disc are joined together by suitable means, as for example a cyanoacrylate adhesive, ultrasonic bond, heat seal bond or radio frequency welding. The barrier disc may include or employ a plurality of leaves having a thin, vapor permeable adhesive on their interior surface. The leaves assist in attaching the first member of the device to the patient.

The wafer seal is adapted for attachment to the barrier disc and/or the first member. The wafer seal ensures and reinforces the bond between the barrier disc and the skin of the patient, as well as allowing conformance of the device to a wide variety of anatomical variations and penile sizes.

The device reduces or eliminates skin maceration, skin irritation, microbial skin infiltration and infection, and urinary tract infection associated with conventional urinary catheters and incontinence devices. To further reduce the risk of microbial infiltration, a microbial barrier layer may be provided on an inner surface of the first member and/or an inner surface of the second member.

The device can be disassembled while on the patient. This feature serves several purposes. The device can remain on the patient when the first and second members are disassembled. Thus, an internal incontinence device can be applied to a patient, and any of the components that follow, such as leg bags or tubing, can be changed without removal of the internal incontinence device. In addition, this design will also allow intermittent internal catheterization when necessary without removal of the external device.

Another preferred embodiment of the invention which is intended to accomplish at least some of the foregoing objects includes an improved two-piece external incontinence device comprising a first member adapted to attach to a parameatal surface of a patient, the first member having a first mating part and a first outlet conduit therethrough to permit fluid to pass therethrough; and a second member having a second mating part and a second outlet conduit therethrough, the second mating part adapted for releasable engagement with said first mating part, the second outlet conduit being in communication with the first outlet conduit to permit fluid to pass from the first outlet conduit to the second outlet conduit and through the second member. The first member is mated to the second member to form an assembly.

The first member may be of such a size and shape to attach comfortably and reliably to seal around the urethral meatus of a patient. The second member has a first end dimensioned to be mated with a straight standard urinary drainage tube or other medical apparatus. The length of the first member facilitates ready, accurate alignment of an outlet conduit, extending through the first and second members, with the meatus. Mating of the first member and the second member is leak-free and reliable, and insures that, when excessive force is applied by pulling on the standard urinary drainage tube, the first and second members separate to eliminate transference of the force to the patient. These mating pieces also allow intermittent internal catheterization without the loss of the parameatal seal.

A preferred method of assembling an external incontinence device in accordance with the invention comprises the steps of providing a first tubular member having a first end and a second end; attaching a first end of the first tubular member to a parameatal surface of a patient; providing a second tubular member having a first end and a second end; providing a drainage tube; attaching the drainage tube to the second end of the second tubular member; and sliding the first end of the second tubular member along the second end of the first tubular member until a mating portion of the second tubular member engages a mating portion of the first tubular member.

Additional objects and advantages of the invention will be set forth in the description which follows, and in part will be obvious from the description, or may be learned by practice of the invention. The objects and advantages of the invention may be realized and obtained by means of the instrumentalities and combinations particularly pointed out in the appended claims.

### BRIEF DESCRIPTION OF THE DRAWINGS

The accompanying drawings, which are incorporated in and constitute a part of the specification. illustrate a presently preferred embodiment of the invention, and, together with the general description given above and the detailed description of the preferred embodiment given below, serve to explain principles of the invention.
Figure 1 is a side elevation view of a two-piece external incontinence device including first and second members and a conventional drainage tube in accordance with the invention;
Figure 2 is a cross-sectional side view of the first member of the two-piece external incontinence device;
Figure 3 is a cross-sectional side view of a first embodiment of the second member of the two-piece external incontinence device;
Figure 4 is a cross-sectional side view of a second embodiment of the second member of the two-piece external incontinence device;
Figure 5 is a cross-sectional side view of the first and second members having an alternative interference fit and locking ring arrangement in accordance with the invention;
Figure 6 is a perspective view disclosing the context of the invention and depicts the two-piece external incontinence device attached to the penis or glans of a patient in accordance with the invention;
Figure 7 is a side perspective view of a second embodiment of the two-piece external incontinence device in accordance with the invention;
Figure 8 is a side perspective view of a second embodiment of the first member of the two-piece external incontinence device; and
Figure 9 is a side perspective view of a third embodiment of the second member of the two-piece external incontinence device.

### DETAILED DESCRIPTION OF THE PREFERRED EMBODIMENTS

The two-piece external incontinence device of the present invention preferably is designed and dimensioned to satisfy a number of considerations. For example, a first member which attaches to the parameatal area of the glans should be of such a size and shape to comfortably and reliably seal around the urethral meatus of a patient. The length of the first member should facilitate easy and accurate visual alignment of the outlet conduit with the meatus. It is preferable that the first member is available in several sizes so that such comfortable and reliable attachment of the first member to any possible size of glans can be made. Testing has shown, however, that a single size for the first member is suitable for a majority of patients. In this preferred embodiment, a wafer seal or strip may be used to over-wrap the device to ensure fit to the patient.

A second member has a first portion which is dimensioned to be mated with the first member and a second portion which is dimensioned to be mated with a standard urinary drainage tube. Mating of the first member and the second member should be leak-free and reliable, but should insure that when excessive force is applied by pulling on a standard urinary drainage tube attached to the second member, the first and second members will separate to eliminate transference of the force to the patient.

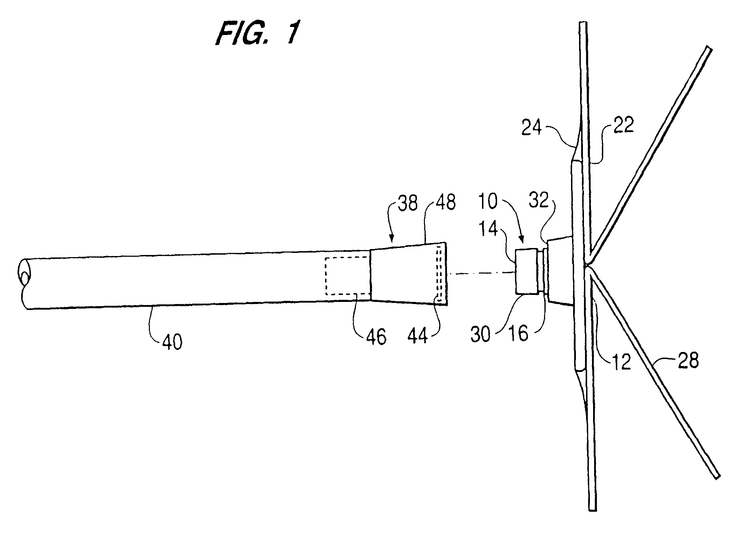

With reference to Figures 1-4, where like numerals indicate like parts, a two-piece external incontinence device is shown which includes a first member, generally indicated 10, and a second member, generally indicated 38. The first member 10 has a first end 12, a second end 14, and an outlet conduit 20 extending therebetween. The overall length of the first member 10 from the first end 12 to the second end 14 is minimized to allow the patient or health care worker, whoever is applying the subject device, or practicing intermittent catheterization through the device, to easily sight the meatus, thus allowing accurate placement of the outlet channel opening with the meatus.

A barrier disc 22 is adhesively mounted to a first end surface 18 of the first member 10. The barrier disc surrounds the outlet conduit 20 at the first end 12 of the first member 10. The barrier disc 22 acts as a microbial barrier layer and protects the skin surface. A relatively small gap remains between the surface 18 of the first end 12 and the parameatal surface of the patient, preventing the first end surface 18 from coming into contact with the parameatal surface, thus avoiding irritation of the parameatal surface.

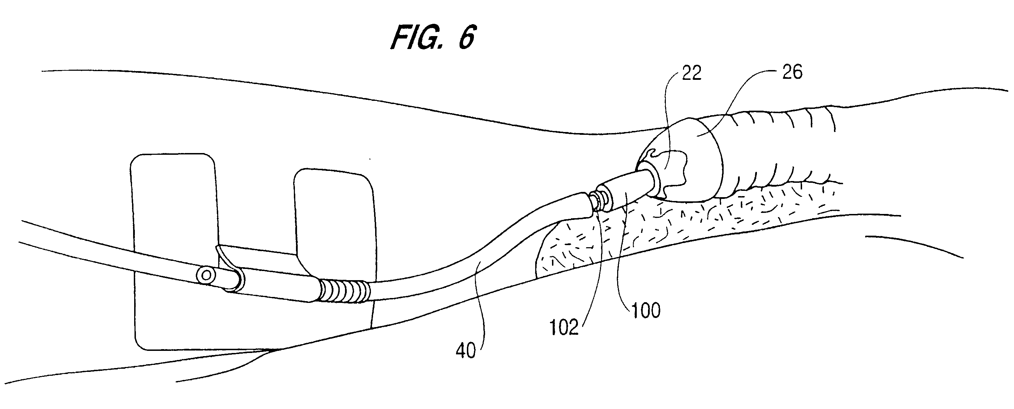

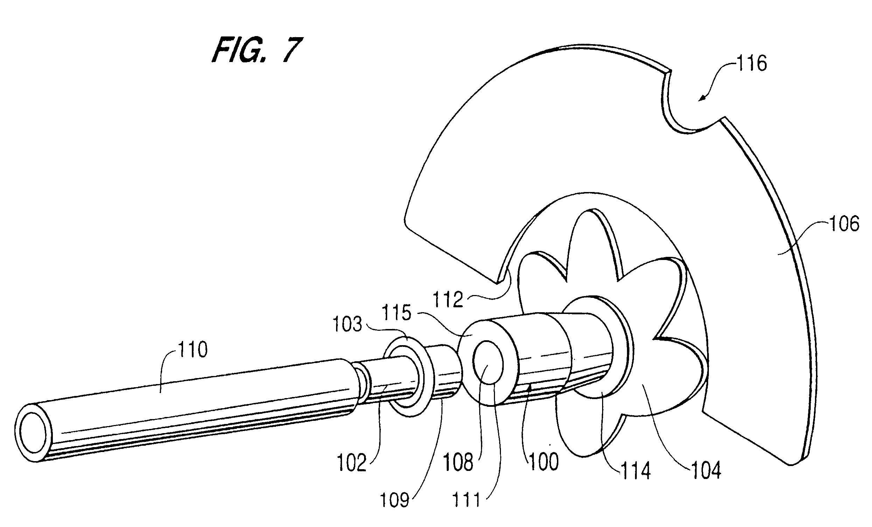

A plurality of leaves 24, shown in Figures 1 and 6, may be applied to the barrier disc 22 at the first end 12 of the first member 10 to secure the first member 10 to the parameatal surface 26 of the patient's penis. The plurality of leaves 24 preferably are composed of a thin film, vapor-transmitting material. The plurality of leaves 24 also may be composed of a hydrocolloid adhesive that can absorb controlled amounts of moisture and may be integral with the barrier disc 22, as shown in Figure 7. The leaves 24 and barrier disc 22 preferably have a protective release backing 28 (shown peeled away from the leaves and the disc in Figure 1) that is removed during application of the device to the skin.

Figure 6 shows the two-piece external incontinence device in context on a male patient. It will be understood by those skilled in the art that the subject invention, suitably sized, may also be used on a female patient. The incontinence device (in this case, the embodiment of Figures 7-9) is shown attached to the parameatal surface 26 of a penis, with the barrier disk 22 and the leaves 24 adhered to the parameatal surface. A drainage tube 40 extends from the device. If the patient should turn and exert force on the tube 40, the two pieces of the device would separate, without transference of uncomfortable, and possibly painful, force to the patient.

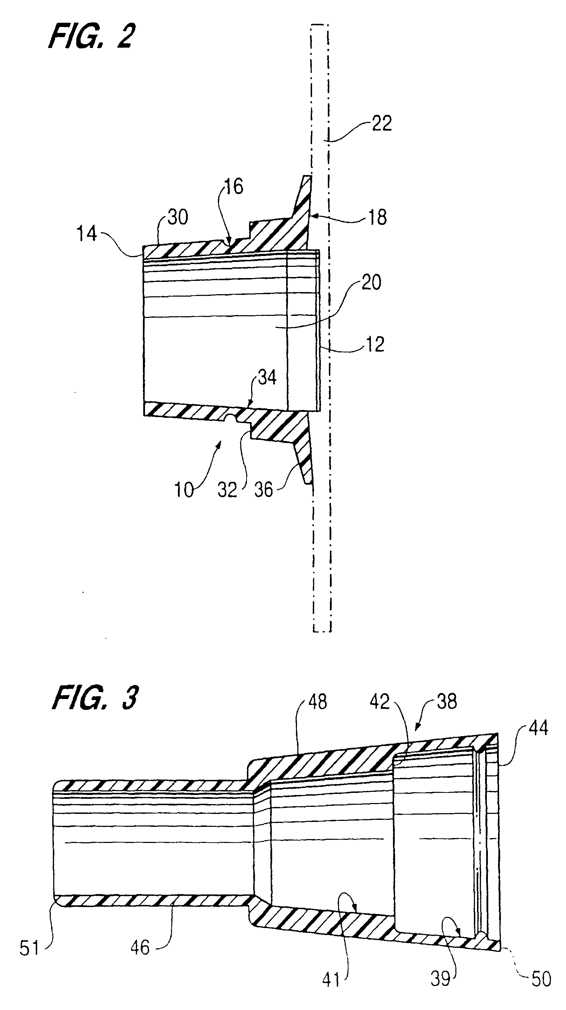

Figure 2 illustrates the first member 10 shown in Figure 1 in detail. The first member includes an outer extension portion 30, an annular shoulder portion 32, and an annular flange portion 36. The first member 10 also has an inner surface 34 which defines the outlet conduit 20. The outlet conduit 20 axially tapers inward at an angle of about five degrees from the first end 12 toward the second end 14 of the first member 10. The first member 10 thus has a reduced diameter at the second end 14 as compared to the first end 12.

The outer extension portion 30, which extends from the second end 14 to the annular shoulder portion 32, includes a mating part 16 (for example, a "locking ring" groove, i.e., a recess, as shown in Figure 2). The mating part of the first member connects with a mating part of the second member 38 to lockingly engage the first and second members of the two-piece external incontinence device, as will be explained below.

Figure 3 shows a first embodiment of the second member 38. The second member 38 includes a first end 50, which mates with the second end 14 of the first member 10, and a second end 51. The second member 38 also includes a generally cylindrical portion 46 and a tapered portion 48 that tapers to have a smaller diameter as it extends farther from the patient.

The tapered portion 48 has a first section defined by an inner surface 39 and has a second section defined by an inner surface 41, separated from the first section by a step 42. The inner diameter along the first section of the second member 38 is essentially the same as the outer diameter along the outer extension portion 30 of the first member 10 such that a tight fit between the first and second members may be established.

In addition, the length of the first surface 39 is approximately equal to the length of the outer extension portion 30 of the first member 10, providing an enhanced fit between the first and second members. Thus, when the first and second members 10 and 38, respectively, are fit together, the first end 50 of the second member 38 abuts the annular shoulder portion 32 of the first member 10.

The second member 38 has a mating part, or "locking ring" raised ridge, 44, i.e., a protrusion. The ridge 44 preferably protrudes inwardly from the inner surface 39 of the first section. The ridge 44 has a height that is approximately the same as the depth of the "locking ring" groove 16. The first end 50 of the second member 38 is adapted to slide over the second end 14 of the outer extension portion 30 of the first member 10 until the ridge 44 of the second member 38 engages the groove 16 of the first member 10. The ridge 44 interacts with (seats in) the groove 16 of the first member 10 to lockingly engage the first and second members of the two-piece external incontinence device and to provide a well fit and secure, yet releasable, locking mechanism.

Placement of the groove and raised ridge may be located at any portion on the inner and outer surfaces of the first and second members as long as the first and second members may matingly engage in a locking arrangement. In addition, it will be understood that the mating portions of the first and second members, i.e., the groove and the ridge, may be continuous or intermittent.

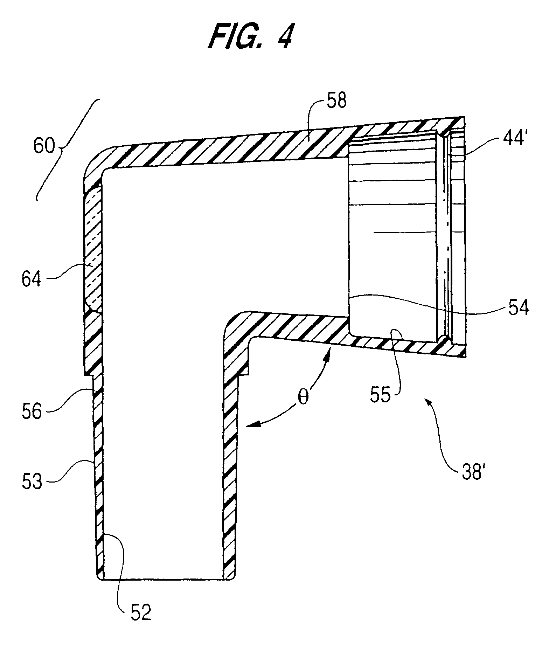

Figure 4 shows a second embodiment of the second member, generally indicated 38'. The second member 38' includes a generally cylindrical portion 56, a tapered portion 58, and an intermediate portion 60 extending therebetween. The generally cylindrical portion 56 has a generally cylindrical inner surface 52 and a slightly angled outer surface 53. The tapered portion 58 has an inner surface 55 including a step 54. The tapered portion 58 also has a mating part, or "locking ring" raised ridge, 44' that outwardly protrudes from the inner surface 55 for connecting to the "locking ring" groove 16 on the outer extension portion 30 of the first member 10. This connection lockingly engages the first and second members of the two-piece external. incontinence device. The first end 62 of the second member 38' is adapted to slide over the second end 14 of the first member 10 until the "locking ring" raised ridge 44' of the second member 38' engages the "locking ring" groove 16 of the first member 10 to engage and lock the first and second members in a secure, sealed assembly.

The raised ridge 44' has a height that is essentially the same as the depth of the "locking ring" groove 16, therefore providing a well-fit and secure, yet releasable, locking mechanism for the first and second members. The inner diameter along the tapered portion 58 of the second member 38' is approximately the same as the outer diameter along the outer extension portion 30 of the first member 10 such that a sufficiently tight fit between the first and second members is established. In addition, the length of the tapered portion 58 to the step 54 approximates the length of the outer extension portion 30 of the first member 10, providing an enhanced fit between the first and second members. Thus, when the first and second members 10 and 38' are fit together, the first end 62 of the second member 38' abuts the annular shoulder portion 32 of the first member 10.

The intermediate portion 60 extends between the generally cylindrical portion 56 and the tapered portion 58. The intermediate portion 60 is shown in Figure 4 to have a curved shape at, for example, a ninety degree angle between the generally cylindrical portion 56 and the tapered portion 58. The intermediate portion 60, however, may be shaped at any angle between one-hundred-and-eighty and ninety degrees, and preferably is shaped between one-hundred-thirty-five and ninety degrees.

This alternate embodiment of the second member 38' may additionally have a molded-in sight window 64, as shown in Figure 4, to facilitate ease of application to the patient by allowing the patient or health care worker, whoever is applying the device, to easily sight the meatus through the previously combined assembly of first and second members 10 and 38' and to obtain accurate alignment of the outlet conduit opening with the meatus. The window 64 is thus transparent and may be located at any position on the second member 38', as long as the window 64 may be used to accurately position the device in place. The positioning of the window 64 depends upon the angle θ.

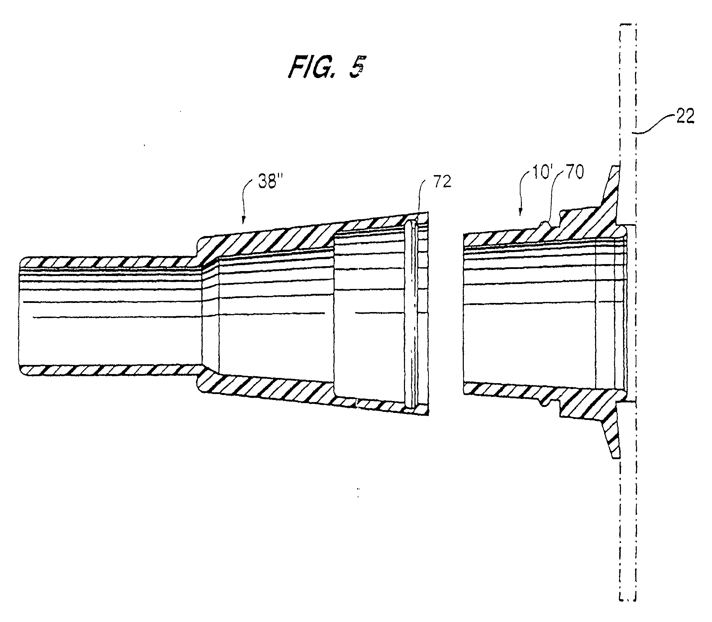

Figure 5 shows a two-piece external incontinence device having an alternative locking arrangement. In this embodiment, the first member 10' has a "locking ring" raised ridge 70 that extends outward from the outer surface of the first member 10'. The second member 38" has a "locking ring" groove 72 located on the inner surface second member 38". The raised ridge 70 seats in the groove 72 upon mating of the first and second members.

Although the preferred disclosed embodiments show a second member that slides on top of a first member, it will be understood that the device may be constructed so that the second member first inside the first member. In such an embodiment, a groove, i.e., recess, may be formed on an inner surface of the first member, and a ridge, i.e., protrusion, may be formed on an outer surface of the second member. Or, a ridge may be formed on the inner surface of the first member, and a ridge may be formed on the outer surface of the second member.

The first member of the two-piece external incontinence device may comprise at least one selected from the group consisting of polyurethane, ethylene-vinyl acetate copolymer, low density polyethylene, or styrene block copolymers, such as Shell Chemical's Kraton, to provide relatively flexible parts that will enhance patient comfort and provide a parameatal seal. The second member of the two-piece external incontinence device may comprise a clear and generally more rigid material, for example, at least one selected from the group consisting of styrene block co-polymers, polyurethane, ethylene-vinyl acetate copolymer, low density polyethylene, crystal polystyrene, polycarbonate or acrylic plastic.

The external incontinence device of the subject invention has been dimensioned such that a mating and leak proof assembly is made when the first and second members are joined. The locking arrangement of the present invention provides at least two novel and distinct advantages: an integral assembly that functions as an 0-ring, thus providing additional security against leaks; and a safety device that provides an audible "click" and tactile feel when the members are mated, thereby reducing or eliminating injury to the patient by eliminating excessive force of application to insure proper seating of the members.

The mating parts are dimensioned so as to ensure that, when excessive force is applied by pulling on a medical device attached to the second end of the second member, such as drainage tube 40 in Figure 6 or a straight connector or other medical apparatus, the first and second members will separate, thereby limiting any transference of the force to the parameatal surface of the patient. This situation is known to occur in a clinical setting, for example, if the patient is in a demented state and pulls or grabs at objects in his immediate environment. It occurs also in spinal cord injury patients, when the drainage tubing may accidentally entwine in the wheels of the chair or other objects in the patient's vicinity.

Figure 7 shows another embodiment of the present invention. This embodiment includes a first member 100, a second member 102, a barrier disc 104, and a wafer seal 106. The first member 100 and the second member 102 are engaged in an interference fit. Thus, the outer diameter of the second member 102 is dimensioned to fit within the outlet channel 108 such that the outer wall 109 of the second member 102 engages the inner wall 111 of the first member 100 in an interference fit. In this embodiment of the invention, the first and second members need not include mating portions in the form of a groove and raised ridge, as seen in the previous embodiments.

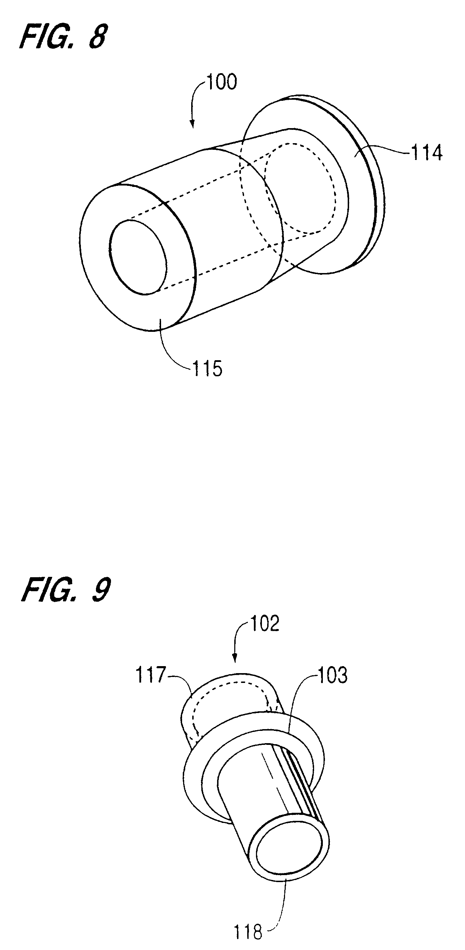

The barrier disc 104 is composed of a hydrocolloid skin adhesive and, preferably, has a plurality of leaves. These leaves may or may not be of the same size. The adhesive barrier disc 104 is attached to a patient's penis or glans and forms a parameatal seal. Attached to the barrier disc 104 is the first member 100 which forms the first part of an outlet channel 108. Figure 8 shows a detailed view of the first member 100. The first member has a flanged first end 114 and a second end 115.

Figure 9 shows a detailed view of the second member 102, which is designed to engage in an interference fit with the first member 100 and to create a tight interference fit with a standard drainage tube or other medical apparatus. The second member 102 has a first end 117 for insertion into the first member 100 and a second end 118. The second member includes also an annular ring 103 that abuts against the second end 115 of the first member 100 when the second member 102 is inserted into the first member 100.

The ability of the first member 100 to be mounted to a patient prior to engagement of the second member 102 with the first member 100 allows accurate placement of the assembly around the patient's meatus. The ability of the first and second member 100 and 102 to separate also allows placement of an intermittent internal catheter without disrupting the parameatal seal. The interference fit between members 100 and 102 is less than between the second member 102 and the drainage tube, thereby allowing the device to separate between members 100 and 102 if, for example, the tube gets caught in a chair wheel or another situation arises which could otherwise transfer a pulling force to the patient.

When the device is positioned on a patient, the wafer seal 106 preferably is wrapped over the barrier disc 104 so that an inner diameter 112 of the seal 106 may abut an outer diameter of the first end 114 of the first member 100. The seal 106 alternatively may partially or entirely overlap the flanged first end 114. The seal 106 wraps over the glans and provides an additional seal with all the same properties as the barrier disc 104. The wafer seal 106 also prevents any possible leakage due to a less than perfect barrier disc seal with the anatomy.

The wafer seal 106 has an optional notch 116 which is useful with uncircumcised males. This notch 116 is fitted around the patient's frenulum and minimizes mechanical irritation in such patients. When laid flat, the seal 106 may resemble a strip, and when mounted to a patient, resembles a truncated cone, with the large part of the cone around the patient's parameatal area and the small part of the cone attached to the barrier disc 104.

Clinical experience with prior external incontinence devices has shown those devices to be subject for additional improvement. For example, when switching from a urinary leg bag to a larger volume night drainage bag or bottle, the drainage tube must be pulled from a standard stepped conical tubing connector, and a new tube must be pushed onto this connector. A great deal of force must be applied to disengage the connector. Further, when a patient purposefully or inadvertently pulls on the drainage tube of an applied incontinence device, a standard stepped conical tubing connector may irreversibly hold the drainage tube to the device housing. The pulling force would then undeniably be transferred to the barrier disc and the parameatal surface of the patient. The present invention provides an incontinence device that enables an improved, simplified, and less time-consuming procedure for switching collection devices.

Moreover, clinical experience with one-piece external incontinence devices identified that it would be desirable to provide a device that allowed simplified sighting through the outlet conduit, in order to optimize accurate alignment of the outlet channel opening with the meatus. Decreasing the length of a one-piece housing to reduce the length of the line of sight was impractical, because properly fitting a drainage hose could not be insured at a length short enough to accomplish easy sighting. After experimentation determined that there was no single length dimension of the housing that would provide both a secure attachment surface for the drainage hose and allow a short enough sight path for easy application, it was determined that a two-piece system, which would meet both objects simultaneously, would be optimum.

Additionally, by temporary disengagement of the first member and the second member, the external incontinence device enables the placement of an intermittent internal catheter, without disrupting the parameatal seal of the device or removal of the device from the patient as would be necessary with the use of a conventional condom catheter.

Further, the subject invention provides a barrier body composed of a limited absorbing hydrocolloid material or a non-absorbing barrier material. For example, the barrier disc may be composed of a polyisobutylene, styrene block copolymer medical pressure sensitive adhesive containing moisture absorbing materials, such as guar gum, carboxymethyl cellulose, gelatin, or blends of the foregoing.

Additional advantages and modifications will readily occur to those skilled in the art. Therefore, the invention in its broader aspects is not limited to the specific details, and representative devices, shown and described herein. Accordingly, various modifications may be made without departing from the scope of the appended claims.

## Claims

1. An external incontinence device comprising:
a first member (10; 10'; 100) adapted to attach to a parameatal surface of a patient, said first member (10; 10'; 100) having a first outlet conduit therethrough to permit fluid to pass therethrough; and
at least one adhesive member (22; 104), operatively connected to said first member (10; 10'; 100), for adhering to the parameatal surface of the patient,
**characterised in that** it further comprises:
a second member (38; 38'; 38"; 102) adapted to releasably engage said first member (10; 10'; 100), said first member (10; 10'; 100) and said second member (38; 38'; 38"; 102) being dimensioned such that said first member (10; 10'; 100) and said second member (38; 38'; 38"; 102) mate in a sealed connection, yet separate when an undesired force is applied to the device, said second member (38; 38'; 38"; 102) having a second outlet conduit therethrough, wherein said second outlet conduit is in communication with said first outlet conduit to permit fluid to pass from said first outlet conduit to said second outlet conduit and through said second member (38; 38'; 38"; 102).

2. The external incontinence device as claimed in claim 1, wherein said first member (10; 10'; 100) has a first mating part and said second member (38; 38'; 38"; 102) has a second mating part, and said first mating part is adapted for releasable engagement with said second mating part.

3. The external incontinence device as claimed in claim 2, wherein said first member (10; 10') and said second member (38; 38'; 38") each have an inner surface, and one of said first mating part and said second mating part comprises a protrusion extending inwardly from said inner surface of a respective one of said first member (10; 10') and said second member (38; 38'; 38").

4. The external incontinence device as claimed in claim 3, wherein said first member (10; 10') and said second member (38; 38'; 38") each have an outer surface, and an other of said first mating part and said second mating part comprises a recess on said outer surface of a respective one of said first member (10; 10') and said second member (38; 38'; 38"), said recess being adapted for releasable engagement with said protrusion.

5. The external incontinence device as claimed in claim 4, wherein said first mating part (16) comprises a recess on said outer surface of said first member (10), and said second mating part (44; 44') comprises a protrusion on said inner surface of said second member (38; 38').

6. The external incontinence device as claimed in claim 5, wherein upon mating of said first member (10; 10') with said second member (38; 38'), said protrusion seats in said recess.

7. The external incontinence device as claimed in claim 2, wherein said first mating part (16; 70) and said second mating part (44; 44'; 72) are annular and form an 0-ring seal between said first member (10; 10') and said second member (38; 38'; 38").

8. The external incontinence device as claimed in claim 6, wherein said protrusion and said recess are dimensioned such that said first member (10; 10') and said second member (38; 38'; 38") are separable by an applied force.

9. The external incontinence device as claimed in claim 2, wherein said first member (10; 10'; 100) further comprises an annular flange (36; 114) extending radially outwardly at a first end of said first member (10; 10'; 100).

10. The external incontinence device as claimed in claim 9, wherein said first member (10; 10') further comprises an annular shoulder (32) extending outwardly between said first mating part (16; 70) and said annular flange (36).

11. The external incontinence device as claimed in claim 10, wherein said second member (38; 38'; 38") further comprises an annular shoulder (42) extending inwardly from an inner surface of said second member (38; 38'; 38").

12. The external incontinence device as claimed in claim 11, wherein upon mating of said first member (10; 10') and said second member (38; 38'; 38"), said annular shoulder (32) of said first member (10; 10') seats against a first end of said second member (38; 38'; 38"), and said annular shoulder (42) of said second member (38; 38'; 38") seats against a second end of said first member (10; 10').

13. The external incontinence device as claimed in claim 4, wherein said first mating part comprises a protrusion on said inner surface of said first member , and said second mating part comprises a recess on said outer surface of said second member.

14. The external incontinence device as claimed in claim 13, wherein upon mating of said first member with said second member, said protrusion seats in said recess.

15. The external incontinence device as claimed in claim 2, wherein said first member (10; 10') and said second member (38; 38'; 38") each have an inner surface, and one of said first mating part and said second mating part comprises a recess on said inner surface of a respective one of said first member (10; 10') and said second member (38; 38'; 38").

16. The external incontinence device as claimed in claim 15, wherein said first member (10; 10') and said second member (38; 38'; 38") each has an outer surface, and an other of said first mating part and said second mating part comprises a protrusion on said outer surface of a respective one of said first member (10; 10') and said second member (38; 38'; 38"), said protrusion adapted for releasable engagement with said recess.

17. The external incontinence device as claimed in claim 16, wherein said first mating part comprises a recess on said inner surface of said first member, and said second mating part comprises a protrusion on said outer surface of said second member.

18. The external incontinence device as claimed in claim 16, wherein said first mating part comprises a protrusion on said outer surface of said first member, and said second mating part comprises a recess on said inner surface of said second member.

19. The external incontinence device as claimed in claim 2, wherein said first mating part (16) comprises an annular recess and said second mating part (44; 44') comprises an annular protrusion.

20. The external incontinence device as claimed in claim 2, wherein said first mating part (70) comprises an annular protrusion and said second mating part (72) comprises an annular recess.

21. The external incontinence device as claimed in claim 2, wherein said second member (38; 38'; 38") comprises a first section on which said first mating part is located, a second section adapted for attachment to a drainage tube (40), and an intermediate section extending between said first section and said second section.

22. The external incontinence device as claimed in claim 21, wherein said intermediate section (60) is bent such that a central axis of said first section (58) extends at an angle between one-hundred-eighty degrees and ninety degrees relative to a central axis of said second section (56).

23. The external incontinence device as claimed in claim 21, wherein said intermediate portion (60) includes a transparent window (64).

24. The external incontinence device as claimed in claim 23, wherein said transparent window (64) comprises at least one selected from the group consisting of crystal polystyrene, polycarbonate, and acrylic plastic.

25. The external incontinence device as claimed in claim 2, wherein said second section member (38') comprises a first section (58) on which said first mating part (16) is located and a second section (56) adapted for attachment to a drainage tube (40), said first section (58) having a central longitudinal axis and said second section (56) having a central longitudinal axis that extends perpendicular to said central longitudinal axis of said first section (58).

26. The external incontinence device as claimed in claim 1, wherein said first member (10; 10'; 100) has a first end, and said at least one adhesive member comprises a barrier disc (22; 22; 104) mounted to said first end of said first member (10; 10'; 100).

27. The external incontinence device as claimed in claim 26, wherein said barrier disc (22; 22; 104) attaches said first member (10; 10'; 100) to the parameatal surface of the patient such that a gap remains between a surface of said first end of said first member (10; 10'; 100) and the parameatal surface of the patient.

28. The external incontinence device as claimed in claim 26, wherein said barrier disc comprises a hydrocolloid adhesive.

29. The external incontinence device as claimed in claim 26, wherein said barrier disc comprises a polyisobutylene, styrene-isoprene-styrene medical pressure sensitive adhesive.

30. The external incontinence device as claimed in claim 1, wherein said first member has a first end and said external incontinence device further comprises at least one leaf mounted to said first end of said first member and to said at least one adhesive member, said at least one leaf having an adhesive surface to mount said first member to a body part of a patient.

31. The external incontinence device as claimed in claim 30, wherein said at least one leaf comprises a vapor-permeable film.

32. The external incontinence device as claimed in claim 1, wherein said first member has an inner surface, and said external incontinence device further comprises a microbial barrier layer disposed on said inner surface.

33. The external incontinence device as claimed in claim 32, wherein said second member has an inner surface, and said external incontinence device further comprises a microbial barrier layer disposed on said inner surface of said second member.

34. The external incontinence device as claimed in claim 1, wherein said first member (10; 10'; 100) comprises at least one selected from the group consisting of styrene block co-polymers, polyurethane, ethylene-vinyl acetate copolymer, and low density polyethylene.

35. The external incontinence device as claimed in claim 1, wherein said second member (38; 38'; 38"; 102) comprises at least one selected from the group consisting of styrene block co-polymers, polyurethane, ethylene-vinyl acetate copolymer, low density polyethylene, crystal polystyrene, polycarbonate, and acrylic plastic.

36. The external incontinence device as claimed in claim 1, wherein said first member (10; 10'; 100) comprises a first material, said second member (38; 38'; 38"; 102) comprises a second material, and said first material is more flexible than said second material.

37. The external incontinence device as claimed in claim 1, wherein said first member (10; 10') tapers in an axial direction from a first end of said first member (10; 10') to a second end of said first member (10; 10').

38. The external incontinence device as claimed in claim 1, wherein said second member (38; 38'; 38") tapers in an axial direction from a first end of said second member (38; 38'; 38") to a second end of said second member (38; 38'; 38").

39. The external incontinence device as claimed in claim 1, wherein said first member (100) and said second member (102) are dimensioned such that said first member (100) and said second member (102) mate in an interference fit.

40. The external incontinence device as claimed in claim 26, and further comprising:
a wafer seal (106) for attachment to at least one of said first end (114) of said first member (100) and said barrier disc (104), said wafer seal (106) being adapted to attach to the parameatal surface of the patient.

41. The external incontinence device as claimed in claim 41, wherein said wafer seal (106) includes a notch (116) on one side thereof.

42. A method of assembling a two-piece external incontinence device as claimed in claim 1, comprising the steps of:
providing a first member (10; 10'; 100) having a first end, a second end, and a mating part;
providing a second member (38; 38'; 38"; 102) having a first end, a second end, and a mating part;
providing a drainage tube (40);
attaching the drainage tube (40) to the second end of the second member (38; 38'; 38"; 102); and
sliding the first end of the second member (38; 38'; 38"; 102) along the second end of the first member (10; 10'; 100) until the mating part of the second member (38; 38'; 38"; 102) engages the mating part of the first member (10; 10'; 100).

43. A method of assembling a two-piece external incontinence device as claimed in claim 1, comprising the steps of:
providing a first member (100) having a first end and a second end;
providing a second member (102) having a first end and a second end;
providing a drainage tube (40);
attaching the drainage tube (40) to the second end of the second member (102); and
engaging the first end of the second member (102) with the second end of the first member (100) in an interference fit.

## Patentansprüche

1. Äußerliche Inkontinenzvorrichtung umfassend:
ein erstes Bauteil (10; 10'; 100) angepasst an die Anbringung an eine Durchgangsoberfläche eines Patienten, wobei das Bauteil (10; 10'; 100) einen ersten Auslasskanal hindurch aufweist, um es Flüssigkeit zu gestatten durch es hindurch zu treten; und
wenigstens ein klebendes Bauteil (22; 104) betreibbar verbunden mit dem ersten Bauteil (10; 10'; 100) zur Anhaftung an die Durchgangsoberfläche des Patienten, **dadurch gekennzeichnet, dass** sie weiter aufweist:
ein zweites Bauteil (38; 38'; 38"; 102) daran angepasst freigebbar in das erste Bauteil (10; 10'; 100) einzugreifen, wobei das erste Bauteil (10; 10'; 100) und das zweite Bauteil (38; 38'; 38"; 102) so bemessen ist, dass das erste Bauteil (10; 10'; 100) und das zweite Bauteil (38; 38'; 38"; 102) sich in einer dichten Verbindung verbinden, sich jedoch voneinander trennen, falls eine unerwünschte Kraft auf die Vorrichtung ausgeübt wird, wobei das zweite Bauteil (38; 38'; 38"; 102) einen zweiten Auslasskanal durch es hindurch hat, wobei dieser zweite Auslasskanal in Verbindung mit dem ersten Auslasskanal steht, um es Flüssigkeit zu gestatten vom ersten Auslasskanal zum zweiten Auslasskanal durch zu treten, und durch das zweite Bauteil (38'; 38"; 102).

2. Äußerliche Inkontinenzvorrichtung nach Anspruch 1, wobei das erste Bauteil (10; 10'; 100) ein erstes Verbindungsteil hat und das zweite Bauteil (38; 38'; 38"; 102) ein Verbindungsbauteil hat und das erste Verbindungsbauteil daran angepasst ist, freigebbar in das zweite Verbindungsbauteil einzugreifen.

3. Äußerliche Inkontinenzvorrichtung nach Anspruch 2, wobei das erste Bauteil (10; 10') und das zweite Bauteil (38; 38'; 38") eine innere Oberfläche aufweisen und eines von dem ersten Verbindungsbauteil und dem zweiten Verbindungsbauteil einen Vorsprung aufweist, der sich inwärts von der inneren Oberfläche eines jeweiligen von dem ersten Bauteil (10; 10') und dem zweiten Bauteil (38; 38'; 38") erstreckt.

4. Äußerliche Inkontinenzvorrichtung nach Anspruch 3, wobei das erste Bauteil (10; 10') und das zweite Bauteil (38; 38'; 38") jedes eine äußere Oberfläche haben und ein anderes von dem ersten Verbindungsbauteil und dem zweiten Verbindungsbauteil eine Vertiefung auf der äußeren Oberfläche von einem jeweiligen aus dem ersten Bauteil (10; 10') und dem zweiten Bauteil (38; 38'; 38") aufweist, wobei die Vertiefung daran angepasst ist, freigebbar in den Vorsprung einzugreifen.

5. Äußerliche Inkontinenzvorrichtung nach Anspruch 4, wobei das erste Verbindungsbauteil (16) eine Vertiefung auf der äußeren Oberfläche des ersten Bauteils (10) aufweist, und das zweite Verbindungsbauteil (44; 44') einen Vorsprung auf der inneren Oberfläche von dem zweiten Bauteil (38; 38') aufweist.

6. Äußerliche Inkontinenzvorrichtung nach Anspruch 5, wobei, während der Verbindung des ersten Bauteils (10; 10') mit dem zweiten Bauteil (38; 38') der Vorsprung sich in die Vertiefung setzt.

7. Äußerliche Inkontinenzvorrichtung nach Anspruch 2, wobei das erste Verbindungsbauteil (16; 70) und das zweite Verbindungsbauteil (44; 44'; 72) ringförmig sind und eine O-Ringdichtung zwischen dem ersten Bauteil (10; 10') und dem zweiten Bauteil (38; 38'; 38") bilden.

8. Äußerliche Inkontinenzvorrichtung nach Anspruch 6, wobei der Vorsprung und die Vertiefung so bemessen sind, dass das erste Bauteil (10; 10') und das zweite Bauteil (38; 38'; 38") durch eine ausgeübt Kraft trennbar sind.

9. Äußerliche Inkontinenzvorrichtung nach Anspruch 2, wobei das erste Bauteil (10; 10'; 100) weiter einen ringförmigen Flansch (36; 114) aufweist, der sich radial auswärts an einem ersten Ende des ersten Bauteils (10; 10'; 100) erstreckt.

10. Äußerliche Inkontinenzvorrichtung nach Anspruch 9, wobei das erste Bauteil (10; 10') weiter eine ringförmige Schulter (32) aufweist, welche sich auswärts zwischen dem ersten Verbindungsbauteil (16; 70) und dem ringförmigen Flansch (36) erstreckt.

11. Äußerliche Inkontinenzvorrichtung nach Anspruch 10, wobei das zweite Bauteil (38; 38'; 38") weiter eine ringförmige Schulter (42) aufweist, die sich inwärts von einer inneren Oberfläche des zweiten Bauteils (38; 38'; 38") erstreckt.

12. Äußerliche Inkontinenzvorrichtung nach Anspruch 11, wobei, während der Verbindung von dem ersten Bauteil (10; 10') und dem zweiten Bauteil (38; 38'; 38") die ringförmige Schulter (32) des ersten Bauteils (10; 10') gegen das erste Ende des zweiten Bauteils (38; 38'; 38") anliegt und die ringförmige Schulter (42) des zweiten Bauteils (38; 38'; 38") gegen das zweite Ende des ersten Bauteils (10; 10') anliegt.

13. Äußerliche Inkontinenzvorrichtung nach Anspruch 4, wobei das erste Verbindungsteil einen Vorsprung auf der inneren Oberfläche des ersten Bauteils aufweist und das zweite Verbindungsteil eine Vertiefung auf der äußeren Oberfläche des zweiten Bauteils aufweist.

14. Äußerliche Inkontinenzvorrichtung nach Anspruch 13, wobei, während der Verbindung von dem ersten Bauteil mit dem zweiten Bauteil der Vorsprung in der Vertiefung sitzt.

15. Äußerliche Inkontinenzvorrichtung nach Anspruch 2, wobei das erste Bauteil (10; 10') und das zweite Bauteil (38; 38'; 38") jeweils eine innere Oberfläche haben und eines aus dem ersten Verbindungsbauteil und dem zweiten Verbindungsbauteil eine Vertiefung auf der inneren Oberfläche eines jeweiligen aus dem ersten Bauteil (10; 10') und dem zweiten Bauteil (38; 38'; 38") aufweist.

16. Äußerliche Inkontinenzvorrichtung nach Anspruch 15, wobei das erste Bauteil (10; 10') und das zweite Bauteil (38; 38'; 38") jedes eine äußere Oberfläche haben und ein anderes aus den ersten Verbindungsbauteil einen Vorsprung aufweist, auf der äußeren Oberfläche von einem jeweiligen aus dem ersten Bauteil (10; 10') und dem zweiten Bauteil (38; 38'; 38"), wobei dieser Vorsprung daran angepasst ist, freigebbar in die Vertiefung einzugreifen.

17. Äußerliche Inkontinenzvorrichtung nach Anspruch 16, wobei das erste Verbindungsbauteil eine Vertiefung auf der inneren Oberfläche des ersten Bauteils aufweist und das zweite Verbindungsbauteil einen Vorsprung auf der äußeren Oberfläche des zweiten Bauteils aufweist.

18. Äußerliche Inkontinenzvorrichtung nach Anspruch 16, wobei das erste Verbindungsbauteil einen Vorsprung auf der äußeren Oberfläche des ersten Bauteils aufweist und das zweite Verbindungsbauteil eine Vertiefung auf der inneren Oberfläche des zweiten Bauteils aufweist.

19. Äußerliche Inkontinenzvorrichtung nach Anspruch 2, wobei das erste Verbindungsbauteil (16) eine ringförmige Vertiefung aufweist und das zweite Verbindungsbauteil (44; 44') einen ringförmigen Vorsprung aufweist.

20. Äußerliche Inkontinenzvorrichtung nach Anspruch 2, bei der das erste Verhindungsbauteil (70) einen ringförmigen Vorsprung aufweist, und das zweite Verbindungsbauteil (72) eine ringförmige Vertiefung aufweist.

21. Äußerliche Inkontinenzvorrichtung nach Anspruch 2, wobei das zweite Bauteil (38; 38'; 38") einen ersten Abschnitt aufweist, auf welchem der erste Verbindungsbauteil angeordnet ist, einen zweiten Abschnitt daran angepasst an eine Drainageröhre (40) angebracht zu werden und einen Zwischenabschnitt, der sich zwischen dem ersten Abschnitt und dem zweiten Abschnitt erstreckt.

22. Äußerliche Inkontinenzvorrichtung nach Anspruch 21, wobei der Zwischenabschnitt (60) gebogen ist, so dass eine zentrale Achse des ersten Abschnitts (58) sich in einem Winkel zwischen 180° und 90° relativ zu einer Zentralachse des zweiten Abschnittes (56) erstreckt.

23. Äußerliche Inkontinenzvorrichtung nach Anspruch 21, wobei der Zwischenabschnitt (60) ein durchsichtlichtes Fenster (64) beinhaltet.

24. Äußerliche Inkontinenzvorrichtung nach Anspruch 23, wobei das durchsichtige Fenster (64) wenigstens eines gewählt aus einer Gruppe bestehend aus Kristallpolystyrol, Polycarbonat und Acrylplastik aufweist.

25. Äußerliche Inkontinenzvorrichtung nach Anspruch 2, wobei das zweite Abschnittsbauteil (38') einen ersten Abschnitt (58) aufweist, auf welchem das erste Verbindungsbauteil (16) angeordnet ist und einen zweiten Abschnitt (56) daran angepasst an eine Drainageröhre (40) angebracht zu werden, wobei der erste Abschnitt (58) eine zentrale Längsachse aufweist und der zweite Abschnitt (56) eine zentrale Längsachse aufweist, die sich senkrecht zur ersten zentralen Längsachse des ersten Abschnitts (58) erstreckt.

26. Äußerliche Inkontinenzvorrichtung nach Anspruch 1, wobei das erste Bauteil (10; 10'; 100) ein erstes Ende hat und wenigstens ein anhaftendes Bauteil eine Sperrscheibe (22; 22; 104) aufweist, die am ersten Ende des ersten Bauteils (10; 10'; 100) angebracht ist.

27. Äußerliche Inkontinenzvorrichtung nach Anspruch 26, wobei die Sperrscheibe (22; 22; 104) das erste Bauteil (10; 10'; 100) an der Durchgangsoberfläche des Patienten anbringt, so dass ein Spalt zwischen einer Oberfläche vom ersten Ende des ersten Bauteils (10, 10'; 100) und der Durchgangsoberfläche des Patienten verbleibt.

28. Äußerliche Inkontinenzvorrichtung nach Anspruch 26, wobei die Sperrscheibe einen Hydrocolloidklebstoff aufweist.

29. Äußerliche Inkontinenzvorrichtung nach Anspruch 26, wobei die Sperrscheibe einen medizinischen druckempfindlichen Polyisobutylen, Styren-Isopren-Styren Klebstoff ausweist.

30. Äußerliche Inkontinenzvorrichtung nach Anspruch 1, wobei das erste Bauteil ein erstes Ende aufweist und die äußerliche Inkontinenzvorrichtung weiter wenigstens eine Blatt aufweist, das am ersten Ende des ersten Bauteils angebracht ist, und am ersten Ende des anhaftenden Bauteils, wobei das wenigstens eine Blatt eine anhaftende Oberfläche aufweist, um das erste Bauteil an einem Körperteil eines Patienten anzubringen.

31. Äußerliche Inkontinenzvorrichtung nach Anspruch 30, wobei das wenigstens eine Blatt einen dampfdurchlässigen Film aufweist.

32. Äußerliche Inkontinenzvorrichtung nach Anspruch 1, wobei das erste Bauteil eine innere Oberfläche aufweist, und die äußerliche Inkontinenzvorrichtung weiter eine mikrobielle Sperrschicht aufweist, die auf der inneren Oberfläche angebracht ist.

33. Äußerliche Inkontinenzvorrichtung nach Anspruch 32, wobei das zweite Bauteil eine innere Oberfläche aufweist, und die äußerliche Inkontinenzvorrichtung weiter eine mikrobielle Sperrschicht aufweist, die auf der inneren Oberfläche des zweiten Bauteils angebracht ist.

34. Äußerliche Inkontinenzvorrichtung nach Anspruch 1, wobei das erste Bauteil (10; 10'; 100) wenigstens eines aufweist, gewählt aus einer Gruppe bestehend aus Styrenblockcopolymeren, Polyurethan, Ethylvinylacetatcopolymer und niedrig dichtem Polyethylen.

35. Äußerliche Inkontinenzvorrichtung nach Anspruch 1, wobei das zweite Bauteil (38; 38'; 38"; 102) wenigstens eines gewählt aus der Gruppe bestehend aus Styrenblockcopolymeren, Polyurethan, Ethylvinylacetatcopolymer, niedrig dichtem Polyethylen, Kristallpolystyrol, Polycarbonat und Acrylplastik aufweist.

36. Äußerliche Inkontinenzvorrichtung nach Anspruch 1, wobei das erste Bauteil (10; 10'; 100) ein erstes Material aufweist, das zweite Bauteil (38; 38'; 38"; 102) ein zweites Material aufweist, und das erste Material flexibler ist als das zweite Material.

37. Äußerliche Inkontinenzvorrichtung nach Anspruch 1, wobei das erste Bauteil (10; 10') sich in einer axialen Richtung von einem ersten Ende des ersten Bauteils (10; 10') zu einem zweiten Ende des ersten Bauteils (10; 10') verjüngt.

38. Äußerliche Inkontinenzvorrichtung nach Anspruch 1, wobei das zweite Bauteil (38; 38'; 38") sich in einer axialen Richtung von einem ersten Ende des zweiten Bauteils (38; 38'; 38") zu einem zweiten Ende des zweiten Bauteils (38; 38'; 38") verjüngt.

39. Äußerliche Tnkontinenzvorrichtung nach Anspruch 1, wobei das erste Bauteil (100) und das zweite Bauteil (102) so bemessen sind, dass das erste Bauteil (100) und das zweite Bauteil (102) sich in einer Presspassung verbinden.

40. Äußerliche Inkontinenzvorrichtung nach Anspruch 26, und weiter aufweisend:
eine Scheibendichtung (106) zur Anbringung an wenigstens einem von dem ersten Ende (114) des ersten Bauteils (100) und der Sperrscheibe (104), wobei die Scheibendichtung (106) daran angepasst ist, an der Durchgangsoberfläche des Patienten angebracht zu werden.

41. Äußerliche Inkontinenzvorrichtung nach Anspruch 40, wobei die Scheibendichtung (106) eine Kerbe (116) auf eine ihrer Seiten beinhaltet.

42. Verfahren zum Zusammenbau an einer zweistückigen äußerlichen Inkontinenzvorrichtung nach Anspruch 1, umfassend die Schritte von:
Bereitstellung eines ersten Bauteils (10; 10'; 100), das ein erstes Ende, ein zweites Ende und einen Verbindungsteil aufweist;
Bereitstellung eines zweiten Bauteils (38; 38'; 38"; 102), das ein erstes Ende, ein zweites Ende und einen Verbindungsteil aufweist;
Bereitstellung einer Drainageröhre (40);
Anbringung der Drainageröhre (40) an dem zweiten Ende des zweiten Bauteils (38, 38 ; 38'; 102); und
Schieben des ersten Endes des zweiten Bauteils (38; 38'; 38"; 102) entlang dem zweiten Ende des ersten Bauteils (10; 10'; 100) bis der Verbindungsteil des zweiten Bauteils (38; 38'; 38"; 102) im Verbindungsteil des ersten Bauteils (10; 10'; 100) eingreift.

43. Verfahren zum Zusammenbau einer zweistückigen äußerlichen Inkontinenzvorrichtung nach Anspruch 1, umfassend die Schritte von:
Bereitstellung eines ersten Bauteils (100), welches ein erstes Ende und ein zweites Ende aufweist;
Bereitstellung eines zweiten Bauteils (102), welches ein erstes Ende und ein zweites Ende aufweist;
Bereitstellung einer Drainageröhre (40);
Anbringung der Drainageröhre (40) an dem zweiten Ende des zweiten Bauteils (102); und
Einrasten des ersten Endes des zweiten Bauteils (102) mit dem zweiten Ende des ersten Bauteils (100) in einer Presspassung.

## Revendications

1. Dispositif d'incontinence externe comprenant :
un premier élément (10 ; 10' ; 100) conçu pour se fixer à une surface paraméatique d'un patient, ledit premier élément (10 ; 10'; 100) ayant une première conduite de sortie à travers celui-ci, pour permettre au fluide de passer à travers celle-ci ; et
au moins un élément adhésif (22 ; 104), connecté de façon opératoire audit premier élément (10 ; 10' ; 100), pour adhérer à la surface paraméatique du patient, **caractérisé en ce qu'**il comprend en outre :
un second élément (38 ; 38' ; 38" ; 102) conçu pour engager de façon libérable ledit premier élément (10 ; 10' ; 100), ledit premier élément (10 ; 10' ; 100) et ledit second élément (38 ; 38' ; 38" ; 102) étant dimensionnés de telle sorte que ledit premier élément (10 ; 10' ; 100) et ledit second élément (38 ; 38' ; 38" ; 102) s'accouplent en une connexion étanche, se séparent cependant lorsqu'une force non désirée est appliquée au dispositif, ledit second élément (38 ; 38' ; 38" ; 102) ayant une seconde conduite de sortie à travers celui-ci, dans lequel ladite seconde conduite de sortie communique avec ladite première conduite de sortie, pour permettre au fluide de passer de ladite première conduite de sortie à ladite seconde conduite de sortie et à travers ledit second élément (38 ; 38' ; 38" ; 102).

2. Dispositif d'incontinence externe selon la revendication 1, dans lequel ledit premier élément (10 ; 10' ; 100) possède une première partie d'accouplement et ledit second élément (38 ; 38' ; 38" ; 102) possède une seconde partie d'accouplement, et ladite première partie d'accouplement est conçue pour un engagement libérable avec ladite seconde partie d'accouplement.

3. Dispositif d'incontinence externe selon la revendication 2, dans lequel ledit premier élément (10 ; 10') et ledit second élément (38 ; 38' ; 38") possèdent chacun une surface interne, et l'une desdites première partie d'accouplement et seconde partie d'accouplement comprend une saillie s'étendant vers l'intérieur à partir de ladite surface interne d'un desdits premier élément (10 ; 10') et second élément (38 ; 38' ; 38") respectif.

4. Dispositif d'incontinence externe selon la revendication 3, dans lequel ledit premier élément (10 ; 10') et ledit second élément (38 ; 38' ; 38") possèdent chacun une surface externe, et une autre desdites première partie d'accouplement et seconde partie d'accouplement comprend un renfoncement sur ladite surface externe d'un desdits premier élément (10 ; 10') et second élément (38 ; 38' ; 38") respectif, ledit renfoncement étant conçu pour un engagement libérable avec ladite saillie.

5. Dispositif d'incontinence externe selon la revendication 4, dans lequel ladite première partie d'accouplement (16) comprend un renfoncement sur ladite surface externe dudit premier élément (10), et ladite seconde partie d'accouplement (44 ; 44') comprend une saillie sur ladite surface interne dudit second élément (38 ; 38').

6. Dispositif d'incontinence externe selon la revendication 5, dans lequel lors de l'accouplement dudit premier élément (10 ; 10') avec ledit second élément (38 ; 38'), ladite saillie se place dans ledit renfoncement.

7. Dispositif d'incontinence externe selon la revendication 2, dans lequel ladite première partie d'accouplement (16; 70) et ladite seconde partie d'accouplement (44 ; 44' ; 72) sont annulaires et forment un joint torique d'étanchéité entre ledit premier élément (10 ; 10') et ledit second élément (38 ; 38' ; 38").

8. Dispositif d'incontinence externe selon la revendication 6, dans lequel ladite saillie et ledit renfoncement sont dimensionnés de telle sorte que ledit premier élément (10 ; 10') et ledit second élément (38 ; 38' ; 38") sont séparables par une force appliquée.

9. Dispositif d'incontinence externe selon la revendication 2, dans lequel ledit premier élément (10 ; 10' ; 100) comprend en outre une collerette annulaire (36 ; 114) s'étendant radialement vers l'extérieur en une première extrémité dudit premier élément (10 ; 10' ; 100).

10. Dispositif d'incontinence externe selon la revendication 9, dans lequel ledit premier élément (10; 10') comprend en outre un épaulement annulaire (32) s'étendant vers l'extérieur entre ladite première partie d'accouplement (16 ; 70) et ladite collerette annulaire (36).

11. Dispositif d'incontinence externe selon la revendication 10, dans lequel ledit second élément (38 ; 38' ; 38") comprend en outre un épaulement annulaire (42) s'étendant vers l'intérieur depuis une surface interne dudit second élément (38 ; 38' ; 38").

12. Dispositif d'incontinence externe selon la revendication 11, dans lequel, lors de l'accouplement dudit premier élément (10; 10') et dudit second élément (38 ; 38' ; 38"), ledit épaulement annulaire (32) dudit premier élément (10 ; 10') se place contre une première extrémité dudit second élément (38 ; 38' ; 38"), et ledit épaulement annulaire (42) dudit second élément (38 ; 38' ; 38") se place contre une seconde extrémité dudit premier élément (10 ; 10').

13. Dispositif d'incontinence externe selon la revendication 4, dans lequel ladite première partie d'accouplement comprend une saillie sur ladite surface interne dudit premier élément, et ladite seconde partie d'accouplement comprend un renfoncement sur ladite surface externe dudit second élément.

14. Dispositif d'incontinence externe selon la revendication 13, dans lequel, lors de l'accouplement dudit premier élément avec ledit second élément, ladite saillie se place dans ledit renfoncement.

15. Dispositif d'incontinence externe selon la revendication 2, dans lequel ledit premier élément (10 ; 10') et ledit second élément (38 ; 38' ; 38") possèdent chacun une surface interne, et l'une desdites première partie d'accouplement et seconde partie d'accouplement comprend un renfoncement sur ladite surface interne d'un desdits premier élément (10 ; 10') et second élément (38 ; 38' ; 38") respectif.

16. Dispositif d'incontinence externe selon la revendication 15, dans lequel ledit premier élément (10 ; 10') et ledit second élément (38 ; 38' ; 38") possèdent chacun une surface externe, et une autre desdites première partie d'accouplement et seconde partie d'accouplement comprend une saillie sur ladite surface externe d'un desdits premier élément (10 ; 10') et second élément (38 ; 38' ; 38") respectif, ladite saillie étant conçue pour un engagement libérable avec ledit renfoncement.

17. Dispositif d'incontinence externe selon la revendication 16, dans lequel ladite première partie d'accouplement comprend un renfoncement sur ladite surface interne dudit premier élément, et ladite seconde partie d'accouplement comprend une saillie sur ladite surface externe dudit second élément.

18. Dispositif d'incontinence externe selon la revendication 16, dans lequel ladite première partie d'accouplement comprend une saillie sur ladite surface externe dudit premier élément, et ladite seconde partie d'accouplement comprend un renfoncement sur ladite surface interne dudit second élément.

19. Dispositif d'incontinence externe selon la revendication 2, dans lequel ladite première partie d'accouplement (16) comprend un renfoncement annulaire et ladite seconde partie d'accouplement (44 ; 44') comprend une saillie annulaire.

20. Dispositif d'incontinence externe selon la revendication 2, dans lequel ladite première partie d'accouplement (70) comprend une saillie annulaire et ladite seconde partie d'accouplement (72) comprend un renfoncement annulaire.

21. Dispositif d'incontinence externe selon la revendication 2, dans lequel ledit second élément (38 ; 38' ; 38") comprend une première section sur laquelle ladite première partie d'accouplement est située, une seconde section conçue pour une fixation à un tuyau de drainage (40), et une section intermédiaire s'étendant entre ladite première section et ladite seconde section.

22. Dispositif d'incontinence externe selon la revendication 21, dans lequel ladite section intermédiaire (60) est pliée de telle sorte qu'un axe central de ladite première section (58) s'étende à un angle entre cent quatre-vingts degrés et quatre-vingt-dix degrés par rapport à un axe central de ladite seconde section (56).

23. Dispositif d'incontinence externe selon la revendication 21, dans lequel ladite partie intermédiaire (60) comprend une fenêtre transparente (64).

24. Dispositif d'incontinence externe selon la revendication 23, dans lequel ladite fenêtre transparente (64) comprend au moins un élément choisi dans le groupe comprenant le polystyrène cristal, le polycarbonate, et le plastique acrylique.

25. Dispositif d'incontinence externe selon la revendication 2, dans lequel ledit second élément de section (38') comprend une première section (58) sur laquelle ladite première partie d'accouplement (16) est située et une seconde section (56) conçue pour une fixation à un tuyau de drainage (40), ladite première section (58) ayant un axe longitudinal central et ladite seconde section (56) ayant un axe longitudinal central qui s'étend perpendiculairement audit axe longitudinal central de ladite première section (58).

26. Dispositif d'incontinence externe selon la revendication 1, dans lequel ledit premier élément (10 ; 10' ; 100) possède une première extrémité, et ledit au moins un élément adhésif comprend un disque-barrière (22 ; 22 ; 104) monté au niveau de ladite première extrémité dudit premier élément (10 ; 10' ; 100).

27. Dispositif d'incontinence externe selon la revendication 26, dans lequel ledit disque-barrière (22 ; 22 ; 104) fixe ledit premier élément (10 ; 10' ; 100) à la surface paraméatique du patient de telle sorte qu'un écart demeure entre une surface de ladite première extrémité dudit premier élément (10 ; 10' ; 100) et la surface paraméatique du patient.

28. Dispositif d'incontinence externe selon la revendication 26, dans lequel ledit disque-barrière comprend un adhésif hydrocolloïde.

29. Dispositif d'incontinence externe selon la revendication 26, dans lequel ledit disque-barrière comprend un adhésif médical sensible à la pression en polyisobutylène, styrène-isoprène-styrène.

30. Dispositif d'incontinence externe selon la revendication 1, dans lequel ledit premier élément possède une première extrémité et ledit dispositif d'incontinence externe comprend en outre au moins une feuille montée au niveau de ladite première extrémité dudit premier élément et dudit au moins un élément adhésif, ladite au moins une feuille ayant une surface adhésive pour monter ledit premier élément sur une partie du corps d'un patient.

31. Dispositif d'incontinence externe selon la revendication 30, dans lequel ladite au moins une feuille comprend un film perméable à la vapeur.

32. Dispositif d'incontinence externe selon la revendication 1, dans lequel ledit premier élément possède une surface interne, et ledit dispositif d'incontinence externe comprend en outre une couche de barrière microbienne disposée sur ladite surface interne.

33. Dispositif d'incontinence externe selon la revendication 32, dans lequel ledit second élément possède une surface interne, et ledit dispositif d'incontinence externe comprend en outre une couche de barrière microbienne disposée sur ladite surface interne dudit second élément.

34. Dispositif d'incontinence externe selon la revendication 1, dans lequel ledit premier élément (10 ; 10' ; 100) comprend au moins un élément choisi dans le groupe comprenant les copolymères séquencés de styrène, le polyuréthanne, le copolymère d'éthylène-acétate de vinyle, et le polyéthylène basse densité.

35. Dispositif d'incontinence externe selon la revendication 1, dans lequel ledit second élément (38 ; 38' ; 38" ; 102) comprend au moins un élément choisi dans le groupe comprenant les copolymères séquencés de styrène, le polyuréthanne, le copolymère d'éthylène-acétate de vinyle, le polyéthylène basse densité, le polystyrène cristal, le polycarbonate, et le plastique acrylique.

36. Dispositif d'incontinence externe selon la revendication 1, dans lequel ledit premier élément (10 ; 10' ; 100) comprend un premier matériau, ledit second élément (38 ; 38' ; 38" ; 102) comprend un second matériau, et ledit premier matériau est plus flexible que ledit second matériau.

37. Dispositif d'incontinence externe selon la revendication 1, dans lequel ledit premier élément (10 ; 10') s'amincit dans une direction axiale à partir d'une première extrémité dudit premier élément (10 ; 10') vers une seconde extrémité dudit premier élément (10 ; 10').

38. Dispositif d'incontinence externe selon la revendication 1, dans lequel ledit second élément (38 ; 38' ; 38") s'amincit dans une direction axiale à partir d'une première extrémité dudit second élément (38 ; 38' ; 38") vers une seconde extrémité dudit second élément (38 ; 38' ; 38").

39. Dispositif d'incontinence externe selon la revendication 1, dans lequel ledit premier élément (100) et ledit second élément (102) sont dimensionnés de telle sorte que ledit premier élément (100) et ledit second élément (102) s'accouplent en un ajustement d'interférence.

40. Dispositif d'incontinence externe selon la revendication 26, et comprenant en outre : un joint d'étanchéité plat (106) pour une fixation à au moins un(e) de ladite première extrémité (114) dudit premier élément (100) et dudit disque-barrière (104), ledit joint d'étanchéité plat (106) étant conçu pour se fixer la surface paraméatique du patient.

41. Dispositif d'incontinence externe selon la revendication 40, dans lequel ledit joint d'étanchéité plat (106) comprend une entaille (116) sur un côté de celui-ci.

42. Procédé d'assemblage d'un dispositif d'incontinence externe en deux parties selon la revendication 1, comprenant les étapes consistant :
à prévoir un premier élément (10 ; 10' ; 100) ayant une première extrémité, une seconde extrémité, et une partie d'accouplement ;
à prévoir un second élément (38 ; 38' ; 38" ; 102) ayant une première extrémité, une seconde extrémité, et une partie d'accouplement ;
à prévoir un tuyau de drainage (40) ;
à fixer le tuyau de drainage (40) à la seconde extrémité du second élément (38 ; 38' ; 38" ; 102) ; et
à faire glisser la première extrémité du second élément (38 ; 38' ; 38" ; 102) le long de la seconde extrémité du premier élément (10 ; 10' ; 100) jusqu'à ce que la partie d'accouplement du second élément (38 ; 38' ; 38" ; 102) engage la partie d'accouplement du premier élément (10 ; 10' ; 100).

43. Procédé d'assemblage d'un dispositif d'incontinence externe en deux parties selon la revendication 1, comprenant les étapes consistant :
à prévoir un premier élément (100) ayant une première extrémité et une seconde extrémité ;
à prévoir un second élément (102) ayant une première extrémité et une seconde extrémité ;
à prévoir un tuyau de drainage (40) ;
à fixer le tuyau de drainage (40) à la seconde extrémité du second élément (102) ; et
à engager la première extrémité du second élément (102) avec la seconde extrémité du premier élément (100) dans un ajustement d'interférence.
